# EUROPEAN PATENT APPLICATION

(11) **EP 3 543 342 A1**
(43) Date of publication of application: **25.09.2019**
(21) Application number: 18162608.6
(22) Date of filing: 19.03.2018
(51) Int. Cl.: C12P 1/04, C12R 1/01, C08L 101/12, B82Y 5/00, B82Y 10/00, B82Y 99/00

(54) **ISOLATED MICROBIAL CONDUCTIVE FIBER MATERIAL**

(71) Applicant: Universiteit Antwerpen, 2000 Antwerpen (BE)
(72) Inventor: MEYSMAN, Filip, 2610 Wilrijk (BE)
(74) Representative: De Clercq & Partners

(57) **Abstract**

Electrically conductive, microbial fibers and fibrous material comprising said fibers, isolated from multicellular cable bacteria filaments and capable of mediating electrical currents over centimetre-scale distances are provided herein. The isolated fibers and fibrous material disclosed herein are suitable for micro- and nano-(bio)electronic applications.

## Description

### FIELD OF THE INVENTION

The present invention relates to electrically conductive, organic fibers that can be isolated from filamentous cable bacteria, to methods to obtain said fibers and to the use of said fibers in micro- and nano-electronic applications.

### BACKGROUND TO THE INVENTION

Micro- and nano-scale electrical conduits are an essential element in micro- and nanoelectronic applications. However, these conduits and their preparation are often prone to significant problems.

Certain microbial materials and structures have been proposed as electrical conductive materials suitable for such applications.
US2012053320 discloses a nanowire comprising a purified protein filament, in particular a pilus, isolated from a bacterium, such as *Geobacter sulfurreducens.* Such a purified pilus contains peptide subunits capable of assembling into the protein filament. The pilus is demonstrated to be electrically conductive, and thus enables to establish an electrical connection, over micrometer distances.
EP2647727 discloses bacteria comprising conductive electronic material made up of bacterial cells which contain conductive fibers. EP2647727 describes that the conductive fibers are arranged in parallel in the periplasmic space along the length and around the periphery of said chain of bacterial cells. However, the document does not disclose isolated fibers nor does it demonstrate the electrical conductivity of the fibers or other properties.

There is a need in the art for conductive material of biological origin suitable for use in nanoelectronics, particularly in bio-nanoelectronics.

### SUMMARY OF THE INVENTION

The present invention relates to organic, electrically conductive fibers which can be obtained from a cable bacterium cell or filament. The present inventors have identified methods of isolating these fibers from these micro-organisms so as to obtain the isolated fibers which are essentially free of cellular structures.

A first aspect of the present invention thus relates to an organic, electrically conductive fiber obtainable from a cable bacteria cell or filament, and to a composition essentially consisting of said fiber. In certain embodiments, said conductive fiber has a diameter between 1 and 150 nm, preferably between 10 and 100 nm, more preferably between 10 and 60 nm. In certain embodiments, said conductive fiber has an electrical conductivity between 0.001 and 100 S cm⁻¹, preferably between 0.1 and 100 S cm⁻¹. In particular embodiments, said fiber is isolated from the multicellular filament of a cable bacterium. Preferably, said cable bacterium belongs to the family of the Desulfobulbaceae, particularly from the genera of Candidatus Electrotrix or Candidatus Electronema.

A related aspect of the present invention provides methods for producing a conductive fiber according to the present invention. These methods allow the generation of a composition essentially consisting of said fiber. In particular embodiments, the methods comprise the steps of:
(i) providing conductive fiber-containing structures isolated from a bacterial cell;
(ii) optionally, washing the fiber-containing structures in a water-alcohol solution, preferably a water-ethanol solution;
(iii) performing subsequent extractions with salt, buffer and buffered sugar solutions, preferably, NaCl, TRIS and sucrose-TRIS solutions;
(iv) incubation in a lysozyme-containing buffer or in a buffer containing another bacterial cell wall degrading enzyme;
(v) collection of the fiber material by centrifugation and removal of the supernatant.
Preferably, said fiber-containing structures are obtained by
(a) Providing bacterial host cells comprising organic conductive fibers;
(b) Contacting the microbial host cells with a detergent, a chelating agent, and/or a bacterial cell wall degrading enzyme degrading the cytoplasm, cell membrane and/or cell wall; and
(c) Separating individual fiber-containing structures from the degraded cell material, such as by physical agitation.
In certain embodiments, said host cell is a native cable bacteria cell, preferably occurring as multicellular bacterial filaments. Preferably, said cable bacteria is from the family of the Desulfobulbaceae, such as from the genus of Candidatus Electrotrix or Candidatus Electronema.

The present invention also relates to an isolated organic, electrically conductive fiber obtainable by the method according to the present invention.

Another aspect of the present inventions relates to the use of the isolated organic, electrically conductive fiber according to the present invention or a composition essentially consisting of said fiber in nano- and micro-electronical applications. In particular embodiments, said fiber or composition essentially consisting of said fiber is provided as a conductive wire or a component thereof.

### BRIEF DESCRIPTION OF THE DRAWINGS

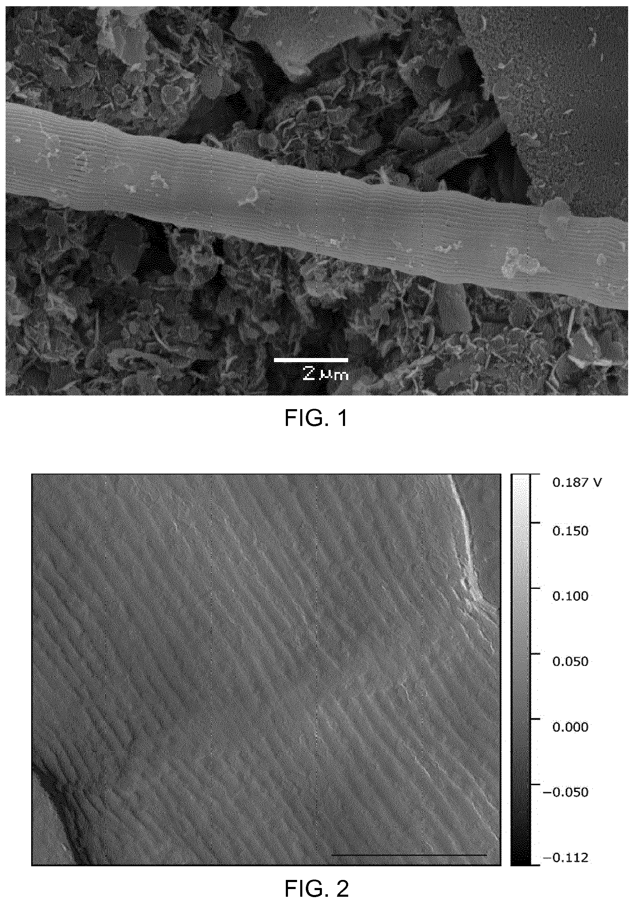

Fig. 1. Scanning electron microscopy (SEM) image of an intact cable bacterium multicellular filament (following the native extraction protocol). The outer surface shows a pattern of parallel ridges, which are continuous along the whole length of the filament.
Fig. 2. Atomic Force Microscopy image of the surface of an intact cable bacterium filament (native extraction protocol).

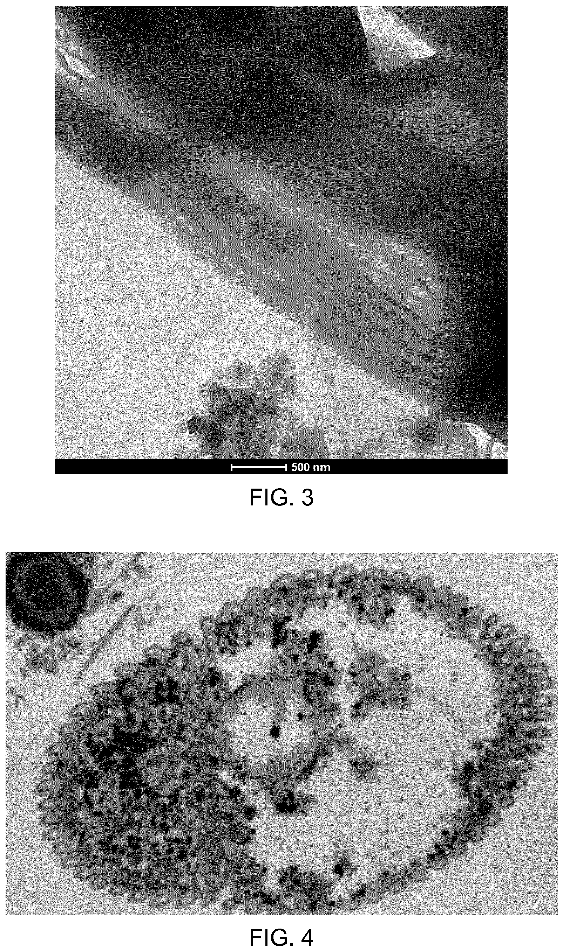

Fig. 3. Detailed image of a physically lysed cable bacterium filament obtained by Transmission Electron Microscopy without any staining or fixation (native extraction protocol). The image reveals that each ridge compartment contains a cylindrical fiber structure, which is electron dense (and hence darkly stained).
Fig. 4. Cross-sectional image of an intact cable bacterium filament obtained by Focused Ion Beam - Scanning Electron Microscopy after resin embedding (native extraction protocol). The cell envelope shows the ridge compartments, which are delineated by membranes (dark grey) and which contain an inner fiber core (light grey).

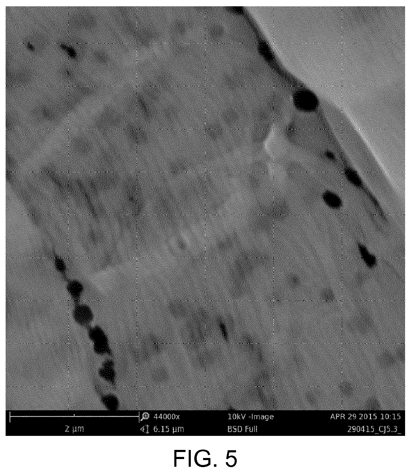

Fig. 5. Scanning Electron Microscopy of a cable bacterium filament obtained via the "cage" extraction protocol. The cage extraction procedure (SDS/EDTA treatment) removes the innner and outer lipid membranes as well as the cytoplasm, creating a translucent cage structure, which contains the periplasmic fibers.

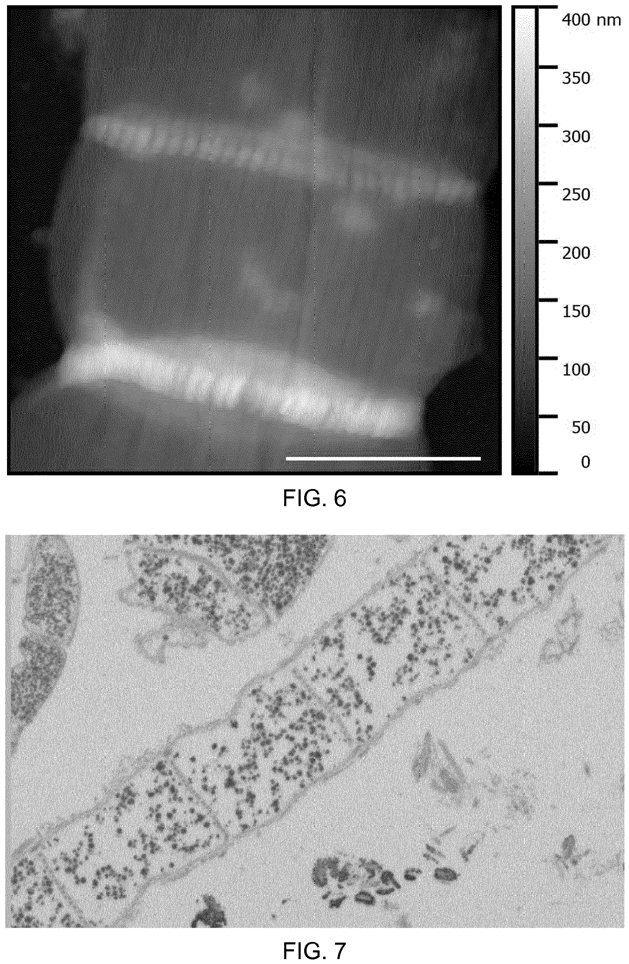

Fig. 6. Atomic Force Microscopy image of a cable bacterium filament treated with the "cage" extraction protocol. The light areas are higher and correspond to the cell junctions.
Fig. 7. Cross-sectional image of a cable bacterium filament obtained via the "cage" extraction protocol. Image obtained by Focused Ion Beam - Scanning Electron Microscopy after resin embedding. The "cage" extraction procedure (SDS/EDTA treatment) removes the innner and outer lipid membranes as well as the cytoplasm (apart from the dark granules).

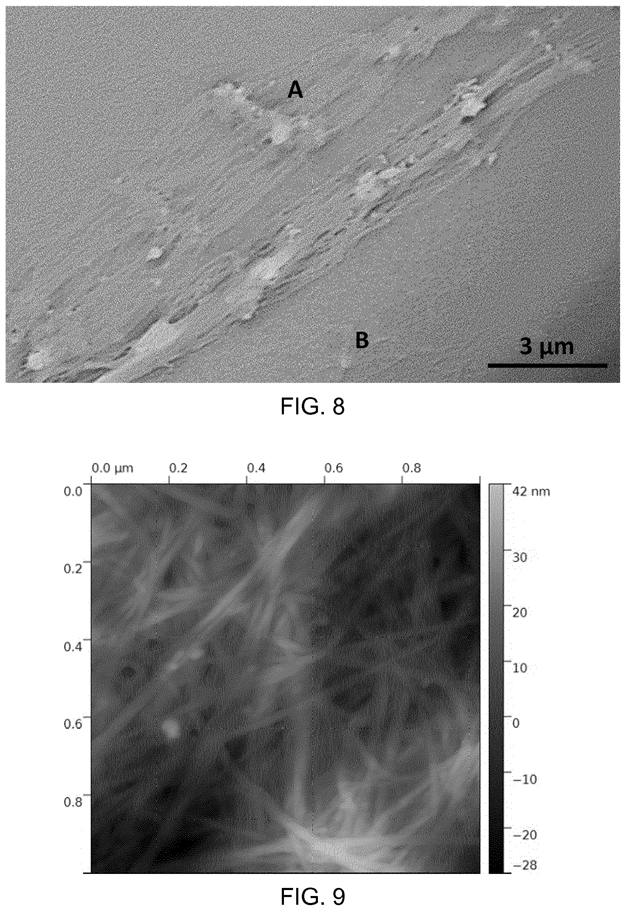

Fig. 8. Scanning Electron Microscopy of cable bacterium filaments that are (partially) digested via the "fiber" protocol. The image reveals the effect of the extraction procedure. When cell junctions are only partially digested, they keep together a parallel bundle of persiplasmic fibers (A). When cell junctions are entirely digested, this gives rise to a mesh of randomly oriented fibers (a so-called "fiber net" (B)). Because of the airy structure, these fiber nets are difficult to distinguish using SEM.
Fig. 9. Atomic Force Microscopy of conductive fiber nets obtained via the "fiber" protocol. The image reveals a random mesh of fiber structures derived from digested cable bacteria filaments.

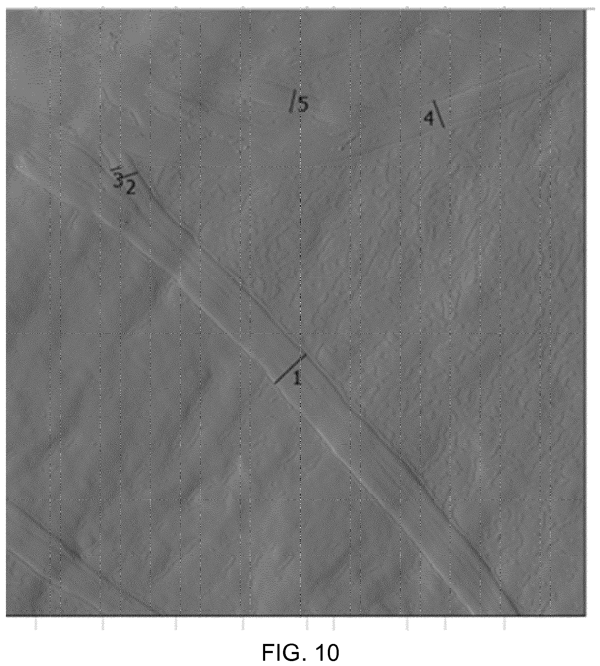

Fig. 10. AFM image of an individual conductive fiber (1) obtained after the extraction procedure with the "fiber" protocol. This image reveals that individual fibers are composed of subfibers (2) and (3).

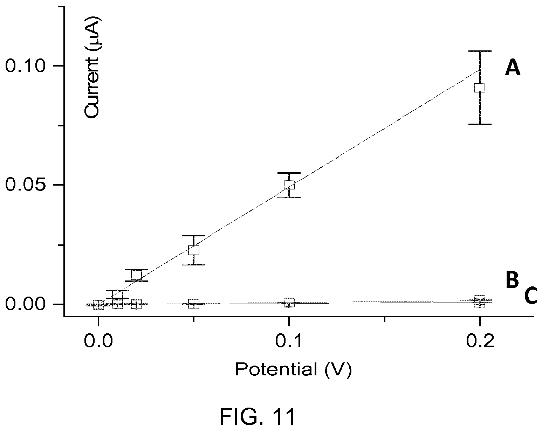

Fig. 11. Current - potential (I-V) curves of intact filaments of cable bacteria (*Electrothrix,* native extraction protocol; upper line A) compared to intact filaments of reference bacteria (*Thiothrix,* native extraction protocol, line B) and the control (no bacteria, line C), showing that intact cable bacteria filaments are highly conductive.

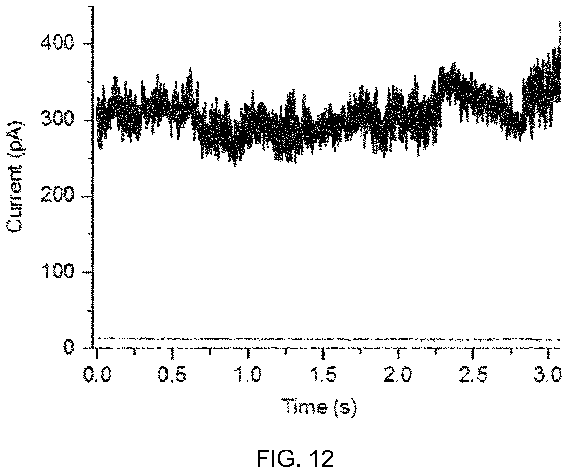

Fig. 12. Current measurements at a fixed bias of 1V for filaments of cable bacteria (*Electrothrix*) extracted via the cage protocol; upper line) compared to the control (no bacteria, bottom line). These results demonstrate that conductive structures must be located inside the periplasmic cage composed of the periplasmic fibers.

### DETAILED DESCRIPTION OF INVENTION

The present invention will be described with respect to particular embodiments but the invention is not limited thereto but only by the claims. Any reference signs in the claims shall not be construed as limiting the scope thereof.
As used herein, the singular forms "a", "an", and "the" include both singular and plural referents unless the context clearly dictates otherwise.
The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" when referring to recited members, elements or method steps also include embodiments which "consist of" said recited members, elements or method steps.
Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.
The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1 % or less of and from the specified value, insofar such variations are appropriate to perform in the disclosed invention. It is to be understood that the value to which the modifier "about" refers is itself also specifically, and preferably, disclosed.
The recitation of numerical ranges by endpoints includes all numbers and fractions subsumed within the respective ranges, as well as the recited endpoints.
All documents cited in the present specification are hereby incorporated by reference in their entirety.
Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may be combined with other embodiments. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. It is to be understood that other embodiments may be utilised and structural or logical changes may be made without departing from the scope of the present invention. The following detailed description, therefore, is not to be taken in a limiting sense, and the scope of the present invention is defined by the appended claims.

The inventors have obtained a novel, naturally conductive, organic material suitable for use in nano-electronic applications, such as for use as an electrically conductive wire. These fibers are of biological origin and can be obtained by isolating fibers and/or a fibrous material comprising said fibers from cable bacteria, particularly from multicellular filaments of cable bacteria.

A first aspect of the present invention provides an organic, electrically conductive fiber, said fiber being obtainable from a cable bacterium cell, preferably obtainable from a multicellular filaments of cable bacteria.
Cable bacteria are long filamentous microorganisms that are capable of conducting electrical currents over centimeter-scale distances. They tend to grow in a chain, thereby forming a chain of cells, called a filament. In such a filament, the conductive fibers are located in the periplasm and run in parallel along the length of the filament. The fibers act as the conduits of electron transport that is necessary for the energy metabolism of the cable bacteria. In particular embodiments, the cable bacteria belong to the candidate genus *Candidatus* Electrothrix or *Candidatus* Electronema.

In particular embodiments, the electrically conductive fiber is an isolated electrically conductive fiber. As used herein, the terms "isolated" or "purified" with respect to a particular component refer to that component being removed from the environment wherein it naturally occurs or wherein it has been synthesized. In particular, an isolated or purified fiber or fibrous material as envisaged herein refers to a fiber or fibrous material which has been removed from the bacterial cell or bacterial filament, particularly from the cell envelope, such as by removing the cytoplasm, lipid membranes and/or other components of the bacterial cell envelope. An isolated or purified fiber according to the present invention is thus essentially free of other bacterial cellular material, more particularly essentially free of bacterial cytoplasm and membranes.

Typically, the diameter of the isolated conductive fiber is within the nanometer scale. In particular embodiments, the isolated organic electrically conductive fiber has a diameter between 1 and 150 nm, preferably between 10 and 100 nm, more preferably between 10 and 60 nm. In specific embodiments, the isolated organic electrically conductive fiber has a diameter of about 10, 15, 20, 25, 30, 35, 40, 45, 50, 55 or 60 nm. Most particularly, the diameters of the fibers are in the range between 10 and 50 nm. Accordingly, in particular embodiments, the composition provided herein consists essentially of isolated fibers having a diameter of 10-50 nm. More particularly, the fibers are not interconnected as bundles. In particular embodiments, the composition comprises a limited number of fibers, such as less than 50, more particularly less than 10. In particular embodiments, the composition comprises one isolated fiber. In particular embodiments, at least some of the fibers in the composition have sub-fibers which branch out from the original fibers, wherein typically the sub-fiber will have a diameter which is reduced in size by 5nm or more than the "parent" fiber.

The length of the isolated conductive fiber can be adapted to the particular application envisaged. In certain embodiments, the length of the isolated conductive fiber depends on the length of the multicellular cable bacteria filament. In certain embodiments, the isolated conductive fiber can be from nm to cm scale, such as from at least 1 µm or 3 µm (corresponding to the length of one cell) to 5 cm, 10 cm or more. In certain embodiments, the conductive fiber has a length between 0.1 µm and 10 cm, such as between 0.01 cm and 5 cm, such as between 0.01 and 1 cm.
However, in certain application, multiple isolated conductive fibers of the present invention may be combined into a conductive material of a longer length. In another certain application, the isolated conductive fibers can be partitioned along the longitudinal axis into smaller sub-fibers, thus providing a conductive fibrous mesh material of smaller diameter sub-fibers.

Advantageously, the conductive fiber according to the present invention is an organic structure with exceptional electrical and conductive properties, which is capable of mediating electrical currents over centimeter scale distances. This renders them suitable for use in bio-inspired electronic devices and nano-electronic applications.
In particular, the conductive fiber according to the present invention has an electrical conductivity between 0.001 and 100 S cm⁻¹, such as between 0.01 and 30 S cm⁻¹ preferably between 0.1 and 10 S cm⁻¹.

Furthermore, the inventors have obtained a conductive fibrous material by removing the cell membranes and cytoplasm of cable bacteria filaments, whereby a so-called ghost cell remains, which consists of parallel fibers that remain tightly connected at the cell junctions, forming a cage-like structure. Depending on the extraction conditions, individual conductive fibers, or fragments thereof, or an organic sheath with fibers embedded was obtained. The present invention thus further provides an isolated conductive material comprising individual conductive fibers according to the present invention, or fragments thereof, or an organic sheath material with conductive fibers embedded, wherein the conductive fibers are interlinked via an organic sheath.

Another aspect of the present invention provides methods for preparing one or more conductive electronic fibers. In particular embodiments, the methods comprise extracting conductive electronic material from bacterial cells.
Suitable bacterial cells in the context of the present invention are bacterial cells from the order of the Desulfobacterales, more particularly from the family of the Desulfobulbaceae. Indeed, some clades within the family of the Desulfobulbaceae are also known as cable bacteria in that they form long multicellular structures capable of electrogenic sulfur oxidation via long-distance electron transport, whereby electrons are transported from cell to cell along the longitudinal axis of the bacterial filaments. Suitable bacteria include bacteria from different genera of the Desulfobulbaceae, such as *Candidatus* Electrothrix and *Candidatus* Electronema. In particular embodiments, the bacterial cell is from the genus Candidatus Electrothrix (Taxon identifier 213121). In particular embodiments, the bacterial cell is an engineered or genetically modified cell of a cable bacterium.

The inventors of the present application for the first time provide methods that allow the production of a single fiber, more particularly by isolation of a single fiber from the periplasmic material of a cable bacteria, or fragments thereof, or an organic sheath with fibers embedded. Accordingly, the methods allow the production of a composition consisting essentially of one or more conductive fibers obtainable from said cable bacteria, or fragments thereof, or an organic sheath with fibers embedded.
The term "essentially consisting of" as used herein when referring to a composition is used to refer to the fact that the composition does not comprise significant amounts of cellular material such as cytoplasm or lipid material originating from the host cell. Typically a composition consisting essentially of one or more fibers will comprise less than 5%, more typically less than 2% w/v of cell material. More particularly, the composition does not comprise any intact cellular structures. In particular embodiments, methods for producing an organic conductive fiber comprise extraction of the conductive fiber from material isolated from a bacterial cell. Conductive material can be isolated from a bacterial cell, more particularly from cells of cable bacteria, in several ways. In particular embodiments, the conductive cell material is isolated by a method comprising the following steps:
(a) Providing a micro-organism comprising organic conductive fibers;
(b) Contacting the micro-organism, subsequently or simultaneously, with a detergent, a chelating agent, and/or a cell wall degrading enzyme, in order to at least partially digest and the cell structures of the micro-organism; and
(c) Separating the conductive material from the cellular structures comprising the cytoplasm, cell membrane and/or cell wall. Such methods for the removal of cellular structures are known in the art and can include mechanic destruction of the cell wall components, such as by physical agitation or a combination of enzymatic and mechanic degradation methods. In particular embodiments, the step of providing a micro-organism comprising organic conductive fibers may comprise culturing or proliferating said micro-organism under conditions suitable for the proliferation of the micro-organism as well as for the production, formation or expression of the conductive fibers, as known by the skilled person.
The present inventors have however identified ways of isolating single fibers from the conductive material of these cable bacteria. These methods involve a number of subsequent extraction steps. In particular embodiments, the methods comprise the steps of:
(i) providing fiber-containing structures from a bacterial cell;
(ii) optionally, washing the fiber-containing structures in a water-alcohol solution; preferably a water-ethanol solution
(iii) performing subsequent extractions with a salt solution, a buffer solution and a buffered sugar solution, preferably a sodium chloride solution, a TRIS solution and a sucrose-TRIS solution;
(iv) incubation in a buffer containing a bacterial cell wall degrading enzyme, preferably incubation in a lysozyme containing buffer;
(v) collection of the fiber material, preferably by centrifugation and removal of the supernatant.

In particular embodiments, the water to alcohol ratio in the water-alcohol solution, preferably water-ethanol solution, ranges between 5:1 and 1:5, preferably ranges between 3:1 or 2:1 and 1:3 or 1:2, more preferably is about 1:1. In particular embodiments, the concentration of the salt solution, preferably sodium chloride solution ranges between 0.1 and 1.0 M, preferably ranges between 0.3 and 0.8 M, more preferably is about 0.5M. In further particular embodiments, the buffer solution or buffered sugar solution, preferably TRIS buffer or sucrose-TRIS buffer, has a buffer concentration, in particular TRIS concentration, ranging between 0.002M and 0.1 M, preferably ranging between 0.005M and 0.05M, more preferably is about 0.01M, and/or has a sugar, in particular sucrose concentration, ranging between 0.1 and 1.0 M, preferably ranges between 0.3 and 0.8 M, more preferably is about 0.5M. In particular embodiments, the concentration of the bacterial cell wall degrading enzyme, in particular lysozyme, ranges between 10mg/ml and 500 mg/ml, preferably between 25mg/ml and 100 mg/ml, more preferably is about 50 mg/ml which is added at a concentration ranging between 0.5% and 5%, preferably ranging between 1% and 3%, more preferably of about 2%.

The methods of the invention allow the production of one or more individual conductive fibers. However, it will be understood that the methods will allow the production of more than one, more particularly a number of fibers from one bacterial cell. Advantageously, these methods provide for the production of uniform organic nanofibers in large quantities.

Another aspect of the present invention provides for the use of the isolated fiber as a conductive material, particularly as nanowires in micro- and nanoelectronic applications and devices. Certain embodiments comprise applying a voltage across one or more conductive fibers.
The present invention further relates to a micro- or nanodimensioned electronic device, an electronic structure, an electronic circuit or an electronic component thereof comprising an isolated conductive fiber according to the present invention. A voltage source, alone or together with one or more suitably-dimensioned circuit components, as would be known in the art, can be connected with or coupled to the one or more conductive fibers according to the present invention in the fabrication of a particular device structure. The electronic components and devices comprising the isolated conductive fiber of the invention may be used in any type of electrical system, which requires the use of conductive fibers. In particular embodiment, the electronic component and/or device is micro- or nano-structured.

Other objects, features, benefits and advantages of the present invention will be apparent to the skilled person from this description and the following exemplary embodiments.

### EXAMPLES

### EXAMPLE 1 - Cultivation and specimen preparation

*Electrothrix* cultivation. Surface sediment was collected from two salt marsh sites in The Netherlands containing cable bacteria of the genus *Candidatus Electrothrix* (Rattekaai, N51°26'21", E04°10'11" and Mokbaai, N53°00'27.1", E4°45'05.1"). Sediments were sieved, homogenized, and incubated at 16°C with oxygenated overlying seawater at *in situ* salinity. The metabolic activity of cable bacteria was monitored through time by O₂, H₂S and pH microsensor depth profiling as described previously (Schauer et al., ISME Journal, 2014, 8, 1314-1322). When sediments showed a clear geochemical fingerprint of electrogenic sulfur oxidation, they were used for cable bacterium filament picking. Individual bacterial filaments or clumps of multiple bacterial filaments were gently expelled from the sediment with a custom-made glass hook needle, and transferred to a drop of purified water (ISO 3696 Grade 1, MilliQ) on a microscope slide. This glass slide was retained in a custom-designed holder allowing on-slide washes and chemical extractions within microvolumes of solvent.

Chemical extractions. Three different extraction protocols were developed and implemented, referred to as "native", "cage" and "fiber" treatments.
In the "native" extraction, surrounding sediment and debris was removed from the outer surface of bacterial filaments, while retaining the integrity of cells and filament structure. To this end, the specimens were exposed to 6 consecutive washes in MilliQ, before being used in the further analysis.
In the so-called "cage" extraction, a partial digestion was performed, which removes the cytoplasm and lipid membranes from the cells of the bacterial filaments, but retains an organic sheath (referred to as "cage") that includes the (conductive) periplasmic fibers. In a first step, the cage treatment involved incubation for 10 minutes at room temperature (RT) in a 1% (w/w) aqueous solution of sodium dodecyl sulfate (SDS), followed by three MilliQ washes. This procedure was repeated 6 times. In a second step, specimen were transferred to a 1mM aqueous solution of ethylene diamine tetra acetic acid (EDTA), incubated for 10 minutes at RT, and subsequently exposed to three MilliQ washes. This procedure was also repeated 6 times.
The so-called "fiber" extraction protocol subjected the samples to an increased digestion, which only retains the perisplasmic fiber structures. To this end, the cytoplasm and lipid membranes were removed (as in the cage protocol), but additionally, the connective material that keeps the fibers together in the fiber cage was digested. The fiber extraction protocol consists of several sequential steps. Bacterial filaments were first transferred into 100 µl of 1:1 vol/vol MilliQ-EtOH solution in a 1.5 ml centrifugation vials (Eppendorf). Subsequently, two NaCI extraction were performed (addition of 100 µl of 0.5 M NaCl, vortexed twice with a 5 min period in between, centrifugation at 10,000 x g for 10 min, removal of the supernatant), followed by a TRIS extraction (addition of 100 µl of 0.01 M Tris-(HCI) buffer at pH = 7.5 vortexed twice with a 5 min period in between, centrifugation at 10,000 x g for 10 min, removal of the supernatant) and a combined sucrose-TRIS extraction (addition of 100 µl 0.5 M sucrose and 100 µl 0.01 M Tris-(HCI) buffer, vortexed twice with a 5 min period in between, centrifugation at 10,000 x g for 10 min, removal of the supernatant). Subsequently, we added 200 µl of 1.0 mM EDTA, 200 µl of 1.0 M Tris-(HCI) buffer and 2 µl of lysozyme (50mg/ml), and the solution was incubated for 30 min on shaker table. The suspension was centrifuged at 10,000 x g for 10 min the supernatant removed.

### EXAMPLE 2 - Microscopy

### Scanning Electron Microscopy (SEM).

Specimen samples were deposited on suitable carriers (aluminium stub coated with a carbon adhesive pad), and subsequently air-dried. The sample was gold-coated for 30 seconds providing a ∼5 nm thick gold layer (Polaron E5100 sputter coater, Van Loenen Instruments, Belgium). Images were collected with a Phenom Pro desktop SEM (Phenom-World B.V., The Netherlands) with a beam intensity of 10 kEV.

### Focused Ion Beam - Scanning Electron Microscopy (FIB-SEM).

Specimen for Focused Ion Beam - Scanning Electron Microscopy (FIB-SEM) were incubated in freshly prepared fixative (2% paraformaldehyde (PFA, Applichem), 2.5% gluteraldehyde (GA, EMS) in 0.15M sodium cacodylate (Sigma-Aldrich) buffer, pH 7.4) at RT for 30 min. Fixative was removed by washing 5 x 3 min in 0.15M cacodylate buffer and samples were incubated in 1% osmium (OsO₄, EMS), 1.5% potassium ferrocyanide (Sigma-Aldrich) in 0.15M cacodylate buffer for 40 min at RT. This was immediately followed by a second incubation in OsO₄ (1% OsO₄ in double-distilled (dd)H₂O) for 40 min at RT. After washing in ddH₂O for 5 x 3 min, samples were incubated overnight at 4°C in 1% uranyl acetate (UA, EMS). The next day, UA was removed by washing in ddH₂O for 5 x 3 min. After final washing steps the samples were dehydrated using ice-cold solutions of increasing EtOH concentration (30%, 50%, 70%, 90%, 2x 100%), for 3 min each. Subsequent infiltration with resin (Durcupan, EMS) was done by first incubating in 50% resin in EtOH for 2 h, followed by at least 3 changes of fresh 100% resin (including 1 overnight incubation). Next, samples were embedded in fresh resin and cured in the oven at 65°C for 72 h.
The resin block containing the samples was mounted on aluminium SEM stubs and samples were coated with ∼6 nm of platinum (Quorum Q150T ES). FIB-SEM imaging was performed using a Zeiss Auriga Crossbeam system. The Focussed Ion Beam (FIB) was set to remove 10nm sections by propelling gallium ions at the surface. In between milling of sections, samples were imaged at 1.5kV using an ESB (back-scattered electron) detector.

Atomic Force Microscopy (AFM). After on-slide chemical treatments, bacterial filaments were placed on mica substrates and left to dry in air. The mica substrates were subsequently glued to stainless steel discs. Images were taken using the Multimode 8 microscope from Bruker in the peak-force Quantum Nano Mechanical (PF-QNM) mode. This is a tapping mode based approach and the piconewton level interaction force is measured directly from the cantilever deflection. The peak force can be maintained as low as 10 pN, which is significantly lower than that used in standard tapping mode (∼1 nN). The probes used during imaging (Scanasyst Air, supplied by Bruker) contain a triangular cantilever with a pyramidal tip, with a nominal tip radius of 2 nm.

Scanning Electron Microscopy (SEM) shows that the cell wall of cable bacteria (after the "native" extraction protocol) exhibits a conspicuous ultrastructure, consisting of parallel ridges that run along the entire longitudinal axis of the filaments (Fig. 1). The parallel ridge structures are also visible via AFM of an intact cable bacterium filament (native extraction protocol; Fig. 2). Detailed analysis of SEM and AFM images shows that cable bacteria filaments can greatly vary in diameter (dB = 0.63 - 2.40 µm) and in the number of parallel ridge structures (NR = 15-54), but that ridge dimensions remain similar across filaments extracted from a range of marine sediments (ridge width: δRW = 144 ± 30 nm). This suggests that cable bacteria construct their cell envelope by the parallel incorporation of a standardized type of ridge component.

Closer inspection reveals that the ridge structures contain fibers, which are visible as electron-dense cylinders in unstained TEM images of physically ruptured cell walls (Fig. 3). These fiber-like structures also extend from underneath the parallel ridges at the damaged end of a filament. A cross-sectional image of an intact cable bacterium filament obtained by Focused Ion Beam - Scanning Electron Microscopy after resin embedding (native extraction protocol) shows the ridge compartments in the cell envelope (periplasm) and which contain an inner fiber core (Fig. 4).

When cell membranes and cytoplasm are entirely removed via the cage extraction protocol, a ghost cell remains. This ghost cell consists of a translucent so-called cage like structure, comprising the parallel fibers, which remain tightly connected at the cell junctions and are glued together by a thin organic sheath (Fig. 5). AFM of a cable bacterium filament treated with the cage extraction protocol also show that the fibers are tightly connected at the cell junctions (visible as light, higher areas; Fig. 6). Focused Ion Beam - Scanning Electron Microscopy provides further details on how the fiber network is embedded in the cell envelope (Fig. 7). Outside the cell junctions, the fiber structures are clearly identifiable in the periplasm underneath the ridges, while within the cell junctions, the fibers remain continuous, but side branches split off radially and converge towards a central node. These star-like connections are also seen when the bacterial filaments are ruptured right at the cell junction. With the cage extraction protocol, a ghost cell remains, confirming that the extraction procedure removes the lipid membranes and the cytoplasm.

When the cable bacteria filaments are digested via the "fiber protocol", only the periplasmic conductive fibers remain (Fig. 8). When the cell junctions are only partially digested, they keep together a parallel bundle of periplasmic fibers. When cell junctions are entirely digested, this gives rise to a mesh of randomly oriented fibers (Fig. 9). AFM of an individual fiber obtained after the "fiber extraction protocol" shows that individual periplasmic fibers are composed of subfibers (Fig. 10). In particular, Fig. 10 shows an individual periplasmic fiber (1) with diameter of 55 nm, composed of subfibers (2) and (3), with diameters of about 27 nm and 12 nm, respectively. Fibers or subfibers (4) and (5) have a diameter of about 37 nm and 29 nm, respectively.

### EXAMPLE 3 - Conductivity of bacterial filaments and fibers

The periplasmic fiber network endows the cell envelope of cable bacteria with a distinct design. This immediately raises the question about the functional role of the fiber network. Without wishing to be bound by theory, the fibers may provide rigidity and structural support to the motile bacterial multicellular filaments, which need to bridge centimeter distances in a dense sediment matrix characterized by substantial contact forces. Indeed, microscopic observations reveal that when filaments are pulled out from the sediment, they can withstand a substantial stress before breaking, and thus the filament must possess a substantial tensile strength. Alternatively, or simultaneously, the fibers act as a conductive "wire network" enabling intercellular electron transport along the longitudinal axis of the bacterial filaments.

Direct electron transport measurements were performed using electrodes with prepatterned golden contact pads with an interdistance between contact pads of Δx = 100 µm (Micrux ED-IDA1-Au). Cable bacteria filaments were retrieved from enrichment cultures, washed in MilliQ, and deposited onto the electrodes. The substrate was subsequently air-dried without any further chemical fixation. Microscopic inspection confirmed that a single filament was present and that the bacterial filament was properly aligned and suitably connected to the golden contact pads. Current-voltage (I-V) data were collected at ambient conditions (Fig. 11). Cable bacterium filaments mediated currents in the range of 1-400 nA at a 2V bias, which translates into corresponding resistances of R = V/I = 5-2800 MΩ. The shape of the I-V curves was highly similar between measurements, with a linear response over the applied voltage range (-0,2V tot + 0.2V), and symmetry between positive and negative bias. For comparison, the same I-V measurements were done with filaments of *Thiothrix flexilis,* a multicellular sulfur-oxidizing bacterium that also produces long filaments, but for which there are no indications of conductive behaviour. Measured currents were 5 orders of magnitude lower. The cross-sectional area of a bacterial filament (3.0 x 10⁻¹² m⁻²) was calculated from the diameter of individual filaments (d_{B} = 2.0 ± 0.1 µm) as determined by SEM imaging, accounting for the flattening of filaments upon drying. Resulting conductivities σ = (I/V)*(Δx/A_{B}) for individual filaments were between 0.01 and 4.4 mS cm⁻¹.

The conductivity of the fibers obtained after subjecting the bacterial multicellular filaments to the "cage extraction protocol" was determined. As discussed above, the "cage extraction protocol" removes the cytoplasm and the membranes of the bacterial filaments, thus leaving behind a denuded fiber network. Nano-ampere currents were measured (Fig. 12). For the specific strains used in the electron transport measurements, FIB-SEM imaging reveals N_{F}= 59-61 fibers per filament, and a mean fiber diameter of δ_{F} = 50 nm, which implies that fibers account for 3.9% of the total cross-sectional area of the cells. With the fibers as the conductive structures, fiber conductivities σ_{F} exceeding 100 mS cm⁻¹ were obtained, which are comparable the values estimated for *Shewanella* nanowires (30 mS cm⁻¹ - Pirbadian et al., ProcNatAcadSci, 2014, 103, 11358-11363) and wild-type Geobacter pili (50 mS cm⁻¹ - Tan et al., Small, 2016, 12, 4481-4485.

### EXAMPLE 4 - Conductivity of filaments and fibers under in vivo conditions

### Currents in filaments and fibers under in vivo conditions

Next, the currents that run through individual filaments and fibers under *in vivo* conditions were estimated. To this end, sediment current densities and cable bacteria abundances from two field surveys and two different laboratory experiments were compiled. Assuming an idealized network, where filaments are oriented vertically and equidistantly spaced in the lateral, the currents supported by individual cable bacterium filaments range from 140 to 370 pA. When translated into an average respiration rate for individual cells (adopting an average cell length of 3 µm), respirational currents of 25-80 fA were obtained that are consistent with bacterial respiration rates measured under environmentally relevant conditions. In dissimilatory metal-reducing bacteria, the respirational currents of individual cells can be guided over micrometer distances towards external solid electron acceptors via pili or nanowires. The current analysis shows that filament currents are at least 4 orders of magnitude higher than nanowire currents in dissimilatory metal reducing bacteria, and moreover, these electrons are transported over distances that are 3 orders of magnitude larger. Such sizeable electron transport over large distances is a direct consequence of the fact the thousands of metabolically active cells are connected to one central conductor network.

While filament currents show a rather broad range, currents become remarkably similar when expressed per individual ridge compartment, or equally per fiber (I_{F} + I_{B}/N_{F}), ranging between 6.2 and 8.0 pA. This similarity in currents across four independent datasets reinforces the idea that the fiber-containing ridge compartments are incorporated as standardized electron transport components in the cell wall of differently sized cable bacteria. Accounting for the cross-sectional area, the estimated currents give rise to current densities ranging between 2600 and 3400 A m⁻².

### Model analysis

To enable electron transport at high such high current densities over centimetre scale-distances, the periplasmic fiber structures must consist of an organic material that is highly conductive. To estimate the electrical conductivity of this material under *in vivo* conditions, we performed a model analysis in which conductive structures are described as a regular cubical stacking of charge localizing sites, where electrons are transferred between neighbouring sites by multi-step electron hopping. Furthermore, no presumptions about the underlying mechanism of electron transfer were made, apart from the constraint that the hopping frequency.
To this ends, four datasets were considered, summarized in Table 1.

**Table 1. A cross-system comparison of electron transport properties of cable bacteria filaments networks - parameters derived from observational data. J = current density in the sediment. N_{B} = cable bacteria filament density. d_{B} = mean filament diameter. ΔL = thickness of the suboxic zone.**

| DATASET | J (mA m⁻²) | N_{B} (10⁸ m⁻²) | d_{B} (µm) | ΔL (mm) |
|---|---|---|---|---|
| Rattekaai Salt Marsh (RSM - field) | 30 | 0.82 | 2.7 | 14 |
| Belgian Coastal Zone Station 130 (BCZ 130 - field) | 25 | 1.2 | 1.2 | 17 |
| Aarhus Bay (AB - laboratory) | 103 | 6.9 | 1.0 | 19 |
| Marine Lake Grevelingen (MLG - laboratory) | 246 | 11 | 1.3 | 14 |

The model analysis was applied to all four datasets specified above, and the results are summarized in Table 2.

**Table 2. A cross-system comparison of electron transport properties of cable bacteria filaments networks - parameters derived from model analysis. I_{F} = current through an individual fiber. δ = charge carrier interspacing. k = electron hopping frequency. σ = electrical conductivity. µ = charge mobility.**

| DATASET | I_{F} (pA) | δ (nm) | k (10¹¹ s⁻¹) | α (%) | σ (S cm⁻¹) | µ (cm²V⁻¹s⁻¹) |
|---|---|---|---|---|---|---|
| RSM | 6.2 | 1.07 | 2.5 | 2.4 | 5.8 | 0.11 |
| BCZ 130 | 7.8 | 1.03 | 3.6 | 5.4 | 6.2 | 0.16 |
| AB | 7.0 | 1.03 | 3.6 | 6.6 | 7.8 | 0.16 |
| MLG | 8.0 | 1.05 | 3.2 | 5.0 | 4.7 | 0.14 |

The model analysis reveals that electron transport through the cell envelope/periplasmic fiber network can explain the observed current densities. However, to do so, the fibers must function as an exceptionally conductive organic structure, with σ = 6.1 ± 1.3 S cm⁻¹. The model-estimated electrical conductivity is by far the highest yet reported for any biological material, and rivals that of moderately doped organic polymers commonly used in photo-voltaic devices. These results all underscore the conclusion that the cable bacteria must have evolved an organic structure with exceptional electrical properties.

### Implications for metabolism

Reactive transport simulations provide support to the hypothesis that the periplasmic fiber network forms the conductive structure. When a detailed biogeochemical model of long-distance electron transport in marine sediments is parameterized with the parameter set for the periplasmic fiber network (δ_{F}, *A_{F},* δ, *k*) discussed here, the model is capable of simulating the characteristic [O₂], [H₂S] and pH depth profiles associated with long-distance electron transport in marine sediments. This data-model comparison shows that the electron flux to and from the conductor, as well as the depth location where this electron exchange takes place, are correctly predicted by the model, and so the overall electron flow through the electron transport network is excellently reproduced. A fiber network with a high conductivity and low ohmic loss provides a selective advantage by enabling a high energy recovery for cellular maintenance and growth in both the cathodic and anodic half-reactions of electrogenic sulphur oxidation. At the same time the model refutes the proposition that the cells may obtain energy from the electron currents that are passing by. Individual cells within multicellular cable filaments are on average 3 µm long, which hence implies that a potential difference of only -0.015 mV exists along the conductor between the terminal ends of the cell. Such a difference in redox potential is way too small to be exploitable for cellular energy conservation. Accordingly, it appears that metabolic energy (e.g. ATP) can be generated by "uploading" and/or "downloading" electrons to the conductive fiber network that spans the length of the cable bacterium filaments, but that cells cannot generate energy from the electrical current that is passing by.

In their broadest sense, our results convincingly demonstrate that the process of long-distance transport in cable bacteria is an exceptional phenomenon of biological charge transfer. The molecular structure and electrical properties of the periplasmic fibers may thus serve as a basis for future, bio-inspired electronic device technology.

## Claims

1. A composition essentially consisting of an organic, electrically conductive fiber obtainable from a cable bacteria cell or filament.

2. The composition according to claim 1, wherein said conductive fiber has a diameter between 1 and 150 nm, preferably between 10 and 100 nm.

3. The composition according to claim 1 or 2 wherein said conductive fiber has an electrical conductivity between 0.001 and 100 S cm⁻¹, preferably between 0.1 and 100 S cm⁻¹.

4. The composition according to any one of claims 1 to 3, wherein said fiber is isolated from a cable bacteria multicellular filament.

5. The composition according to any one of claim 1 to 4, wherein said cable bacteria belong to the family of the Desulfobulbacea.

6. The composition according to any one of claim 1 to 4, wherein said cable bacteria is from the genus of Candidatus Electrotrix or Candidatus Electronema.

7. A method for producing an organic conductive fiber or a composition essentially consisting of said fiber according to any of claims 1 to 6 comprising the steps of:
(i) providing fiber-containing structures isolated from a bacterial cell;
(ii) optionally, washing the fiber-containing structures in a water-alcohol solution;
(iii) performing subsequent extractions with a salt solution, a buffer solution, and a buffered sugar solution, preferably a NaCl, TRIS and sucrose-TRIS solution;
(iv) incubation in a buffer containing a bacterial cell wall degrading enzyme, preferably lysozyme; and
(v) collection of the fiber material by centrifugation and removal of the supernatant.

8. The method of claim 7, wherein said fiber-containing structures are obtained by
(a) Providing bacterial host cells comprising organic conductive fibers;
(b) Contacting the microbial host cells with a detergent, a chelating agent, and/or a bacterial cell wall degrading enzyme degrading the cytoplasm, cell membrane and/or cell wall;
(c) Separating individual fiber-containing structures from the degraded cell material.

9. The method according to claim 7 or 8, wherein said host cell is a native cable bacteria cell, preferably wherein said cable bacteria occur as multicellular bacterial filaments.

10. The method according to claim 7 to 9, wherein said cable bacteria is from the family of the Desulfobulbaceae.

11. The method according to any one of claims 7 to 10, wherein said cable bacteria is from the genus of Candidatus Electrotrix or Candidatus Electronema.

12. An isolated organic, electrically conductive fiber obtainable by the method of any one of claims 7 to 11.

13. Use of the composition according to any of claims 1 to 6 or the isolated organic, electrically conductive fiber according to claim 12 in nano- and micro-electronical applications.

14. The use according to claim 13, wherein said fibers are provided as a conductive wire or a component thereof.
